# EUROPÄISCHE PATENTSCHRIFT

(11) **EP 1 311 233 B1**
(45) Veröffentlichungstag und Bekanntmachung des Hinweises auf die Patenterteilung: **08.04.2009**
(21) Anmeldenummer: 01971943.4
(22) Anmeldetag: 21.08.2001
(51) Int. Cl.: A61K 9/113

(54) **SUBSTANZEN ZUR INDUKTION UND INTENSIVIERUNG DER BRÄUNUNGSMECHANISMEN DER HAUT UND KOSMETISCHE ODER DERMATOLOGISCHE FORMULIERUNGEN DIESELBEN ENTHALTEND**
SUBSTANCES FOR INDUCING AND INTENSIFYING THE TANNING MECHANISMS OF THE SKIN AND COSMETIC OR DERMATOLOGICAL PREPARATIONS WHICH CONTAIN SAID SUBSTANCES
SUBSTANCES PERMETTANT D'INDUIRE ET D'INTENSIFIER LES MECANISMES DE BRONZAGE DE LA PEAU ET FORMULATIONS COSMETIQUES OU DERMATOLOGIQUES CONTENANT CES SUBSTANCES

(30) Priorität: 23.08.2000 DE 10041272
(43) Veröffentlichungstag der Anmeldung: 21.05.2003
(73) Patentinhaber: Beiersdorf AG, 20245 Hamburg (DE)
(72) Erfinder: BLATT, Thomas, 22559 Hamburg (DE); BERENS, Werner, 22307 Hamburg (DE); STÄB, Franz, 21379 Echem (DE); WOLBER, Rainer, 22399 Hamburg (DE)
(86) Internationale Anmeldenummer: PCT/EP2001/009655
(87) Internationale Veröffentlichungsnummer: WO 2002/015861

(56) Entgegenhaltungen:
- FR-A- 2 596 986
- US-A- 4 749 573
- DATABASE WPI Section Ch, Week 198949 Derwent Publications Ltd., London, GB; Class B04, AN 1989-362798 XP002187681 & SU 1 462 198 A (MOSC SECOND MED INS), 28. Februar 1989 (1989-02-28)

## Beschreibung

Die vorliegende Erfindung betrifft Substanzen zur Induktion und Intensivierung der Bräunungsmechanismen der Haut und kosmetische oder dermatologische Formulierungen dieselben enthaltend.

Unter künstlicher Bräunung sind alle Maßnahmen zu verstehen, die der Haut ohne Einwirkung von Sonnenstrahlen ein gebräuntes Aussehen verleihen. Dazu sind verschiedene Möglichkeiten prinzipiell denkbar, wie beispielsweise
■ physikalische Anfärbung durch Anwendung von Schminken oder Make-up,
■ Anfärbung durch chemische Reaktionen mit Hilfe von selbstbräunenden Zubereitungen,
■ Bräunung durch (orale) Einnahme von Carotinoiden sowie
■ künstliche Bestrahlung unter Verwendung technisch hergestellter Lichtquellen.
   Dabei ist zu beachten, daß es bei der Bräunung mit ultravioletter Strahlung, wie sie im natürlichen Sonnenspektrum vorkommt oder von künstlichen Lichtquellen erzeugt wird, bei zu langer Einwirkung oder Überschreitung einer bestimmten Dosis zu nicht unerheblichen Hautschäden kommen kann.
   Setzt man die Haut zu lange der Sonne oder einer künstlichen Strahlenquelle aus, so entwickelt sich nach einer Latenzzeit von 2 bis 3 Stunden eine gegen die unbestrahlte Haut stark abgegrenzte Hautrötung, das Erythema solare. Bei dem so entstehenden Sonnenbrand unterscheidet man zwischen
■ 1. Grad: Erythem (Rötung, Wärmegefühl)
   klingt nach 2 bis 3 Tagen wieder ab und verschwindet unter gleichzeitig zunehmender Pigmentierung,
■ 2. Grad: Blasenbildung
   auf der Haut bilden sich Blasen mit Brennen und Jucken, die Oberhaut wird flächig abgestoßen,
■ 3. Grad: Zellschädigung
   es treten tiefgehende Zellschädigungen auf, der Körper reagiert mit Fieber, die Oberhaut wird großflächig abgestoßen.
Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm.

Etwa 90 % der auf die Erde gelangenden ultravioletten Strahlung bestehen allerdings aus UV-A-Strahlen mit einer Wellenlänge zwischen 320 nm und 400 nm. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Man hat lange Zeit fälschlicherweise angenommen, daß die langwellige UV-A-Strahlung nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

Moderne Bräunungsanlagen, wie sie beispielsweise in Solarien verwendet werden, produzieren überwiegend UV-A-Strahlung in hoher Bestrahlungsstärke. Verglichen mit der natürlichen Mittagssonne im sommer liegt die erzeugte UV-A-Bestrahlungsstärke in der Regel um den Faktor 3 bis 5 höher, in Einzelfällen sogar bis zum Faktor 10. Gleichzeitig wird ein geringer Anteil an UV-B-Strahlen ausgesandt. Das Strahlenspektrum ist daher in der Regel in bezug auf die Bräunung günstiger und hinsichtlich des Sonnenbrandrisikos weniger bedenklich als das natürliche Sonnenspektrum.

Der entscheidende Vorteil von Solarien ist die Möglichkeit, eine genaue und begrenzte Bestrahlungsdosis bei definierter Strahlenqualität anzuwenden, was unter natürlichen Bedingungen kaum gegeben ist. Selbstverständlich ist aber insbesondere infolge der erhöhten UV-A-Bestrahlungsstärke ein gewisses Risiko nicht auszuschließen.

Dementsprechend sollte, wer lichtbedingte Hautschäden wie Sonnenbrand, chronische Lichtschäden oder ein mögliches Karzinomrisiko vermeiden will, aber andererseits auf die modisch erwünschte Bräune nicht verzichten möchte, auf solche Methoden der Bräunung zurückgreifen, die ohne die Anwendung künstlich erzeugter oder natürlicher ultravioletter Strahlen auskommen.

Die einfachste Art und Weise, seiner Haut einen braunen Farbton zu verleihen, ist das Auftragen entsprechend gefärbter Schminken oder Make-up-Präparate. Allerdings werden selbstverständlich nur solche Körperpartien angefärbt, die von den farbigen Präparaten überdeckt werden. Ein Nachteil der Schminken ist deshalb die zeitraubende Prozedur des Auftragens. Ferner nachteilig ist, daß sie stark auf Textilien wie Hemdkragen oder Blusen abfärben. Darüber hinaus können die verschiedenen Farbstoffe unterschiedliche allergene Potenz aufweisen und sogar hautirritierend wirken.

Eine weitere Möglichkeit zur künstlichen Bräunung ist, Substanzen auf die Haut aufzutragen, die mit der Hornschicht eine chemische Farbreaktion eingehen, wobei es zu einer artifiziellen Bräunung der obersten Hautschichten kommt. Der wichtigste und nach wie vor am häufigsten eingesetzte Wirkstoff in selbstbräunenden Zubereitungen (sog. "Selbstbräunern") ist das Dihydroxyaceton (DHA), ein im menschlichen Körper vorkommender dreiwertiger Zucker. DHA reagiert mit den Proteinen und Aminosäuren der Homschicht, wobei der Farbton über eine Polymerisation zustande kommt. Der entstehende Farbton sowie die Intensität der Verfärbung sind von der Art der Aminoverbindungen abhängig und werden beispielsweise auch durch die Zubereitung und die Dicke der Homschicht beeinflußt.

Diese Art der Bräunung ist eine rein chemische Reaktion der Hornschicht. Hierdurch wird zwar ein gewisses kosmetisches Ergebnis erreicht, jedoch schilfert die Bräune recht schnell wieder ab und ist ohne jede Schutzfunktion für die Haut. Diese Bräunung ist völlig unabhängig von den Melanozyten der Haut, die die natürliche Hautbräunung durch das von ihnen gebildete Pigment Melanin herbeiführen.

Die Anfärbung durch Selbstbräuner erfolgt ohne Sonnenlichteinwirkung. Im Gegensatz dazu werden auch sogenannte Pre-Tan-Produkte oder Tan-Promoter angeboten, die vor der Sonnenbestrahlung aufgetragen werden müssen In der Sonne tritt dann eine Vergilbung dieser Zubereitungen ein, die zu einer leichten Braungelb-Färbung der Oberhaut führen soll, welche die "Sonnenbräune" zusätzlich verstärkt.

Eine weitere, von UV-Licht ebenfalls völlig unabhängige Art der künstlichen Bräunung kann durch die Hormone herbeigeführt werden, die im Körper auch infolge (natürlicher) UV-Bestrahlung üblicherweise freigesetzt werden und die Melanozyten letztlich zur Melanin-Synthese anregen. Zu nennen wären in diesem Zusammenhang beispielsweise Abkömmlinge von Proopiomelanocortin (POMC) wie aMSH und synthetische Varianten (wie NDP), die zum Teil weitaus höhere Aktivität als das natürliche aMSH aufweisen. Zwar kann durch diese Hormone grundsätzlich eine Bräunung herbeigeführt werden, jedoch verbietet sich ihr Einsatz in Kosmetika, da es sich eindeutig um pharmakologisch wirkende Substanzen (Hormone) handelt, welche nicht ohne medizinische Indikation breit eingesetzt werden sollten.
Aus SU 1 462 198 A sind Fettemulsionen enthaltend Lipofuscin C zur intravenösen Applikation bekannt.
Aufgabe der vorliegenden Erfindung war es also, die Nachteile des Standes der Technik zu vermeiden und Substanzen zur Bräunung der Haut finden, durch deren Anwendung der Haut auch ohne Einwirkung von Sonnenstrahlen ein gebräuntes Aussehen verliehen wird und welche die vorhandene natürliche Bräunung der Haut intensivieren und die natürliche Hautbräunung länger anhalten lassen.

Insbesondere sollten diese Substanzen gesundheitlich unbedenklich, nicht reizend und leicht zu handhaben sein und der resultierende Farbton der natürlichen gesunden Hautfarbe entsprechen. Die erhaltene Bräunung sollte lichtecht und nicht abwaschbar sein.

Eine weitere Aufgabe der vorliegenden Erfindung war es, kosmetische oder dermatologische Zubereitungen zur Bräunung der Haut zu finden. Es war überraschend und darin liegt die Lösung dieser Aufgaben, daß den Nachteilen des Standes der Technik abgeholfen wird durch
die Verwendung einer oder mehrerer Substanzen, welche gewählt werden
aus der Gruppe der Lipofuscine, zur Bräunung der Haut.

Lipofuscine im Sinne der vorliegenden Erfindung entstehen im Rahmen natürlicher Prozesse innerhalb der menschlichen Zellen. Lipofuscin ist ein heterogenes, kohlenhydrat-, protein- und cholesterin- bzw. lipidhaltiges, gelbliches bis braunes Pigment, das in Form von Granula im Plasma verschiedener Körperzellen vorkommt. Lipofuscine treten v. a. im Alter als sogenanntes Abnutzungspigment z. B. in Nerven- und Herzmuskelzellen auf. Lipofuscin-Granula werden wahrscheinlich aus Lysosomen gebildet, in denen sich nicht weiter verwertbare Zellbestandteile angesammelt haben. Im Alter reichem sie sich im Cytoplasma somit auch in der Haut an.

Erfindungsgemäße Lipofuscine sind im Rahmen von lipidperoxidativen Prozessen erhältlich; die mehrstufigen Reaktionen laufen unter anderem über Malonaldehyde, die mit primären Aminen über Schiff'sche Base-Reaktionen zur Bildung von Lipofuscinen führen.

Vorteilhafte Ausgangsstoffe für derartige Reaktionen sind Aldehyde, welche aus lipidperoxidativen Prozessen stammen, wie beispielsweise Malonaldehyde und Malodialdehyde. Diese reagieren bevorzugt mit Substanzen, welche Aminogruppen enthalten, wobei letztere vorteilhaft ebenfalls aus Lipidkomponenten stammen. Ferner vorteilhaft kann die freie Aminogruppe auch aus N-terminalen Enden von (Lipo-) Proteinen und Peptiden stammen. Besonders vorteilhafter Ausgangsstoff sind Phosphatidylethanolamin, Sphingosin sowie Dihydrosphingosin und deren Homologen mit verschieden langen (ungesättigten) Acylresten.

Bevorzugt enthalten erfindungsgemäße Lipofuscine mindestens eine stickstoffhaltige Ringstruktur.

Erfindungsgemäße Lipofuscine sind ferner z. B. erhältlich durch artifizielle chemische (z. B. mit Wasserstoffperoxid) oder physikalische (durch Einwirkung von UV-A-Strahlung) Oxidation von Liposomen - insbesondere von Liposomen, welche Phosphatidylethanolamin enthalten - und/oder durch Oxidation von isolierten, zellulären Organellen (Mitochondrien) und/oder Membranen.

Ferner vorteilhaft im Sinne der vorliegenden Erfindung sind Lipofuscine, welche sich durch die folgende Strukturformel auszeichnen: 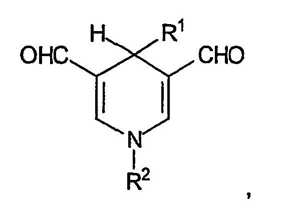 worin R¹ und R² - je nach Wahl der Ausgangsstoffe - unabhängig voneinander unterschiedlichste Reste darstellen können, vorteilhaft aber Peptid-, Aminosäure- oder Proteinreste. Es kann sich gleichermaßen um Phospholipide (Phosphatidylethanolamin) oder Sphingosine oder Dihydrosphingosine bzw. Bestandteile daraus handeln.

Weiterhin vorteilhaft im Sinne der vorliegenden Erfindung sind Lipofuscine, welche sich als lineare Kondensationsprodukte durch Schiffsche Base-Reaktionen aus primären Aminen und Malondialdehyd (R¹-N=CH-CH=CH-N-R²) oder Hydroxyketosen ableiten, worin R¹ und R² - je nach Wahl der Ausgangsstoffe - unabhängig voneinander unterschiedlichste Reste darstellen können, vorteilhaft aber Peptid-, Aminosäure- oder Proteinreste. Es kann sich gleichermaßen um Phospholipide (Phosphatidylethanolamin) oder Sphingosine oder Dihydrosphingosine bzw. Bestandteile daraus handeln.

Die bräunenden Substanzen im Sinne der vorliegende Erfindung zeichnen sich u.a. dadurch aus, daß sie - nach topischer Applikation - in der Haut die Bildung von haupteigenen Pigmenten induzieren, die Melaninsynthese steigern und auf diese Weise eine verstärkte Bräunung der Haut erzeugen. Sie sind gesundheitlich unbedenklich, nicht reizend und leicht zu handhaben, und der resultierende Farbton entspricht naturgemäß dem der natürlichen gesunden Hautfarbe. Die erhaltene Bräunung ist - da sie der natürlichen Bräunung entspricht - lichtecht und nicht abwaschbar. Durch die erfindungsgemäßen Substanzen wird ferner überraschend die Bräunung bereits gebräunter Haut verstärkt und darüber hinaus das Bleichwerden gebräunter Haut verzögert.

Ein weiterer Vorteil der vorliegenden Erfindung ergibt sich aus den Schutzeigenschaften von in der Haut gebildetem natürlichem Melanin. Neben verschiedenen weiteren Funktionen des hauteigenen Melanins (wie beispielsweise "Entgiftung" bzw. Bindung von toxischen Substanzen und/oder Pharmaka etc.), sind ferner insbesondere diese Funktionenen von Melanin für die Haut sehr wichtig, u.a. in Bezug auf die Homeostase, die Vermeidung der Hautalterung und dergleichen:
Melanin wirkt als natürlicher UV-Filter zum Schutz vor schädigenden UV-Strahlen sowie darüberhinaus als Antioxidanz zum Schutz vor reaktiven Sauerstoffspezies (oxidativem Stress), die unter anderem durch Sonnenbestrahlung auftreten können.

Somit ergibt sich bei erfindungsgemäßer Verwendung (d. h. nach topischer Applikation des erfindungsgemäßen Wirkstoffs) nicht nur ein kosmetischer Nutzen im Sinne einer verstärkte Bräunung durch die gesteigerte Melanin-Synthese in der Haut sondern auch ein zusätzlicher Nutzen durch die verschiedenen Schutzleistungen von Melanin.

Dementsprechend eigenen sich die erfindungsgemäßen Substanzen insbesondere hervorragend als bräunende Wirkstoffe in kosmetischen oder dermatologischen Zubereitungen.

Gegenstand der vorliegenden Erfindung sind daher auch
kosmetische oder dermatologische Formulierungen, dadurch gekennzeichnet, daß sie mindestens eine Substanz enthalten, welche gewählt wird aus der Gruppe der Lipofuscine.

Die erfindungsgemäßen Substanzen und kosmetische oder dermatologische Zubereitungen dieselben enthaltend induzieren in der Haut die Bildung von hauteigenen Pigmenten, intensivieren die vorhandene natürliche und/oder künstliche Bräunung der Haut und lassen die Hautbräunung länger anhalten.

Die erfindungsgemäßen Formulierungen sind in jeglicher Hinsicht überaus befriedigende Präparate, die sich durch eine gleichmäßig färbende Wirkung auszeichnen. Es war für den Fachmann nicht vorauszusehen gewesen, daß die erfindungsgemäßen Formulierungen
■ einfacher zu formulieren sein,
■ der Haut schneller und besser ein natürlich gebräuntes Aussehen verleihen,
■ die Hautbräunung länger anhalten lassen,
■ besser als feuchtigkeitsspendende Zubereitungen wirken,
■ besser die Hautglättung fördern,
■ sich durch bessere Pflegewirkung auszeichnen,
■ bessere sensorische Eigenschaften, wie beispielsweise die Verteilbarkeit auf der Haut oder das Einzugsvermögen in die Haut, aufweisen und
■ einen besseren/risikolosen Eigenschutz der Haut (vor UV-Strahlung) bieten würden
als die Zubereitungen des Standes der Technik. Zudem entfalten die erfindungsgemäßen Formulierungen überraschenderweise keine Hormonwirkungen.

Kosmetische oder dermatologische Formulierungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Emulsionen können vorzugsweise auch eine Mikroemulsion, eine Pickering-Emulsion oder eine sprühbare Emulsion sein.

Darüber hinaus können die erfindungsgemäßen Formulierungen aber auch vorteilhaft in Form von ölfreien Zubereitungen, wie beispielsweise Gelen, oder als wasserfreie Zubereitungen vorliegen.

Vorteilhaft ist es, den Gehalt an einer oder mehreren Substanzen aus der Gruppe der Lipofuscine in der kosmetischen oder dermatologischen Formulierung zwischen 0,0001 und 30 Gew.-%, besonders vorteilhaft zwischen 0,01 und 10 Gew.%, ganz besonders zwischen 0,1 und 2 Gew.-% zu wählen, jeweils bezogen auf das Gesamtgewicht der Formulierung.

Die erfindungsgemäßen Formulierungen können ferner vorteilhaft auch Dihydroxyaceton oder Nußextrakte enthalten sowie weitere Substanzen, welche die Bräune erhalten oder erzeugen sollen.

Die erfindungsgemäßen Formulierungen können vorteilhaft, wenngleich nicht zwingend, auch in Kombination mit UV-Strahlung - sei es mit künstlich erzeugten oder natürlichen ultravioletten Strahlen - verwendet werden, beispielsweise um die natürliche Bräunung noch zu steigern oder aber um eine besonders lang anhaltende Bräunung zu erreichen. Hierzu können die erfindungsgemäßen Zubereitungen vorteilhaft z. B. vor, nach oder während der Einwirkung von UV-Strahlen auf die Haut aufgebracht werden, beispielsweise in Form einer Presun-, einer Sonnenschutz- oder einer Aftersunformulierung.

Die erfindungsgemäßen kosmetischen und/oder dermatologischen Formulierungen können wie üblich zusammengesetzt sein und insbesondere zur Behandlung und der Pflege der Haut und/oder der Haare, als Schminkprodukt in der dekorativen Kosmetik oder als Lichtschutz- bzw. sogenanntes Pre- oder Aftersunpräparat dienen. Entsprechend können die erfindungsgemäßen Formulierungen - je nach ihrem Aufbau - beispielsweise verwendet werden als Hautschutzcrème, Gesichtscrème, Reinigungsmilch, Sonnenschutzlotion, Nährcrème, Tages- oder Nachtcrème usw.

Zur Anwendung werden die erfindungsgemäßen kosmetischen und dermatologischen Formulierungen in der für Kosmetika üblichen Weise auf die Haut und/oder die Haare in ausreichender Menge aufgebracht.

Die erfindungsgemäßen kosmetischen und dermatologischen Formulierungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel und/oder Silikonderivate sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Homschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure und/oder ein fucosereiches Polysaccharid, welches in den Chemical Abstracts unter der Registratumummer 178463-23-5 abgelegt und z. B. unter der Bezeichnung Fucogel®1000 von der Gesellschaft SOLABIA S.A. erhältlich ist.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Camosin, D-Camosin, L-Camosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Ferner ist es vorteilhaft, wenn die kosmetischen oder dermatologischen Formulierungen im Sinne der vorliegenden Erfindung Enzyme bzw. Enzym-Mimics enthalten, die als enzymatische Antioxidantien wirken, wie z. B. Superoxiddismutasen, Katalasen, Peroxidasen, Selenoproteine.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.
   Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.
   Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.
   Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.
   Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.
   Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.
   Von den Kohlenwasserstoffen sind Paraffinöl, Squalan und Squalen vorteilhaft im Sinne der vorliegenden Erfindung zu verwenden.
   Vorteilhaft kann die Ölphase ferner einen Gehalt an cyclischen oder linearen Silikonölen aufweisen oder vollständig aus solchen Ölen bestehen, wobei allerdings bevorzugt wird außer dem Silikonöl oder den Silikonölen einen zusätzlichen Gehalt an anderen Ölphasenkomponenten zu verwenden.
   Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).
   Besonders vorteilhaft sind ferner Mischungen aus Cyclomethicon und Isotridecylisononanoat, aus Cyclomethicon und 2-Ethylhexylisostearat.
   Die wäßrige Phase der erfindungsgemäßen Formulierungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi und/oder Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z. B. Fe₂O₃), Zirkoniums (ZrO₂) Siliciums (SiO₂), Mangans (z. B. MnO), Aluminiums (Al₂O₃), Cers (z. B. Ce₂O₃), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz sowie das 1,4-di(2-oxo-10-Sulfo-3-bornylidenmethyl)-Benzol und dessen Salze (besonders die entsprechenden 10-Sulfatoverbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnen wird.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate. Insbesondere bevorzugt sind das 2,4-Bis-{[4-(2-Ethyl-hexyloxy)-2-hydroxy]-phenyl}-6-(4-methoxyphenyl)-1,3,5-triazin (lNCl: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Besonders bevorzugte UV-Filtersubstanz im Sinne der vorliegenden Erfindung ist ferner ein unsymmetrisch substituiertes s-Triazin, welches auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-pheny}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl) 1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B-Filter können öllöslich oder wasserlöslich sein. Vorteilhafte öllösliche UV-B-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester,
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.
   Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Herstellungsbeispiel für Lipofuscin

Leber-Gewebe wird in 0,3M Sucrose auf Eis homogenisiert und anschließend für 10 Minuten bei 270 g (1g steht hier und im nachfolgenden für die einfache Erdbeschleunigung) zentrifugiert. Der Überstand hieraus wird ein weiteres mal bei 1100 g über 20 Minuten zentrifugiert, das entstehende Sediment verworfen und der Überstand abermals zentrifugiert (diesesmal 10 Minuten bei 3000 g). Das aus der letzten Zentrifugation verbleibende Sediment, das überwiegend aus Mitochondrien besteht, wird ins PBS (Phosphate Bufferd Saline, ein einfacher Phosphat-Puffer bei pH 7,4) aufgenommen und wie zuvor zentrifugiert (Waschschritt). Das verbleibende, von Sucrose weitgehend gereinigte Pellet wird in 2ml PBS aufgenommen und unter der sterilen Werkbank 20 Stunden mit UV-Licht bestrahlt. Während dieser Zeit findet die Lipidperoxidation (u.a. Entstehung von Malondialdehyd) statt, was über Schiff'sche Basen-Reaktionen mit freien Aminogruppen (aus Proteinen und Phosphatidylethanolamin der Mitochondrien-Membranen) zur Bildung von Lipofuscin führt.

Das Lipofuscin wird in den nachfolgenden Rezepturbeispielen eingesetzt.

### Beispiel 1

### W/O Creme

| | Gew.-% |
|---|---|
| Paraffinöl (DAB 9) | 10,00 |
| Petrolatum | 4,00 |
| Wollwachsalkohol | 1,00 |
| PEG-7-Hydriertes Rizinusöl | 3,00 |
| Aluminiumstearat | 0,40 |
| Glycerin | 2,00 |
| Konservierungsmittel, Farbstoffe, Parfüm | q.s. |
| Lipofuscin | 0,20 |
| Wasser | ad 100,00 |

### Beispiel 2

### W/O Lotion

| | Gew.-% |
|---|---|
| Paraffinöl (DAB 9) | 20,00 |
| Petrolatum | 4,00 |
| Glucosesesquüsostearat | 2,00 |
| Aluminiumstearat | 0,40 |
| Phytoen | 1,00 |
| Glycerin | 5,00 |
| Konservierungsmittel, Farbstoffe, Parfüm | q.s. |
| Lipofuscin | 2,50 |
| Wasser | ad 100,00 |

### Beispiel 3

### O/W Lotion

| | Ges.-% |
|---|---|
| Paraffinöl (DAB 9) | 8,00 |
| Isopropylpalmitat | 3,00 |
| Petrolatum | 4,00, |
| Cetylstearylalkohol | 2,00 |
| PEG 40 Rizinusöl | 0,50 |
| Natriumcetylstearylsulfat | 0,50 |
| Natrium Carbomer | 0,40 |
| Glycerin | 3,00 |
| α-Glucosylrutin | 0,20 |
| Octylmethoxycinnamat | 5,00 |
| Butylmethoxydibenzoylmethan | 1,00 |
| Konservierungsmittel, Farbstoffe, Parfüm | q.s. |
| Lipofuscin | 0,02 |
| Wasser | ad 100,00 |

### Beispiel 4

### O/W Creme

| | Gew.-% |
|---|---|
| Paraffinöl (DAB 9) | 7,00 |
| Avocadoöl . | 4,00 |
| Glycerylmonostearat | 2,00 |
| Natriumlactat | 3,00 |
| Glycerin | 3,00 |
| Konservierungsmittel, Farbstoffe, Parfüm | q.s. |
| Lipofuscin | 0.4 |
| Wasser | ad 100,00 |

### Beispiel 5

### Liposomenhaltiges Gel

| | Gew.-% |
|---|---|
| Lecithin | 6,00 |
| Schibutter | 3,00 |
| Taurin | 0,20 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Konservierungsmittel, Farbstoffe, Parfüm | q.s. |
| Lipofuscin | 1,20 |
| Wasser | ad 100,00 |

### Beispiel 6

### Sonnenschutzemulsion

| | Gew.-% |
|---|---|
| Cyclomethicon | 2,00 |
| Cetyldimethicon Copolyol | 0,20 |
| PEG 22-Dödecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| Lipofuscin | 0,50 |
| D-Biotin | 0,50 |
| ZnSO₄ | 0,70 |
| Na₄EDTA | 0,30 |
| Konservierungsmittel, Farbstoffe, Parfüm | q.s. |
| Wasser | ad 100,00 |

### Beispiel 7

### Sonnenschutzemulsion

| | Gew.-% |
|---|---|
| Cyclomethicon | 2,00 |
| Cetylstearylalkohol + PEG 40-hydriertes Rizinusöl + | 2,50 |
| Natrium Cetylstearylsulfat Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,50 |
| Lipofuscin | 0,20 |
| α-Tocopherylacetat | 1,00 |
| Na₃HEDTA | 1,50 |
| Konservierungsmittel, Farbstoffe, Parfüm | q.s. |
| Wasser | ad 100,00 |

### Beispiel 8

### Sonnenschutzemulsion

| | Gew.-% |
|---|---|
| Cyclomethicone | 2,00 |
| Cetylstearylalkohol +PEG 40-hydriertes Rizinusöl | 2,50 |
| +Natrium Cetylstearylsulfat | |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,75 |
| Na₃HEDTA | 1,50 |
| Konservierungsmittel, Farbstoffe, Parfüm | q.s. |
| Lipofuscin | 3,00 |
| Wasser | ad 100,00 |

### Beispiel 9

### Sprayformulierung

| | Gew.-% |
|---|---|
| Ubiquinon 10 | 0,10 |
| Lipofuscin | 0,80 |
| Ethanol | 28,20 |
| Konservierungsmittel, Farbstoffe, Parfüm | q.s. |
| Propan/Butan 25/75 | ad 100,00 |

### Beispiel 10

### O/W Creme

| | Gew.-% |
|---|---|
| Paraffinöl (DAB 9) | 7,00 |
| Soyaöl | 4,00 |
| Glycerylmonostearat | 2,00 |
| Natriumlactat | 3,00 |
| Glycerin | 8,00 |
| Konservierungsmittel, Farbstoffe, Parfüm | q.s. |
| Lipofuscin | 0,08 |
| Wasser | ad 100,00 |

## Patentansprüche

1. Verwendung einer oder mehrerer Substanzen, welche gewählt werden aus der Gruppe der Lipofuscine zur nicht-therapeutischen Bräunung der Haut.

2. Verwendung einer oder mehrerer Substanzen, welche gewählt werden aus der Gruppe der Lipofuscine zur nicht-therapeutischen Steigerung der Melaninsynthese in der Haut.

3. Verwendung einer oder mehrerer Substanzen, welche gewählt werden aus der Gruppe der Lipofuscine zur nicht-therapeutischen Verstärkung der vorhandenen Bräunung der Haut.

4. Verwendung einer oder mehrerer Substanzen, welche gewählt werden aus der Gruppe der Lipofuscine zur nicht-therapeutischen Verlängerung der Braunfärbung der Haut.

5. Verwendung einer oder mehrerer Substanzen, welche gewählt werden aus der Gruppe der Lipofuscine, zur Herstellung einer kosmetischen oder dermatologischen Zubereitung zur Bräunung der Haut, zur Steigerung der Melaninsynthese, zur Verstärkung der vorhandenen Bräunung und/oder zur Verlängerung der Braunfärbung der Haut.

6. Verwendung einer oder mehrerer Substanzen, welche gewählt werden aus der Gruppe der Lipofuscine, zum nicht-therapeutischen Schutz der Haut vor oxidativem Stress.

7. Verwendung einer oder mehrerer Substanzen, welche gewählt werden aus der Gruppe der Lipofuscine, zur Herstellung einer kosmetischen oder dermatologischen Zubereitung zum nicht-therapeutischen Schutz der Haut vor oxidativem Stress.

8. Verwendung nach einem der vorhergehenden Ansprüche, **dadurch gekennzeichnet, dass** das oder die Lipofuscine aus der Gruppe gewählt werden, welche gebildet wird aus 1-Amino-3-Iminopropen sowie aus den N,N'-substituierten Derivaten und 1,4-Dihydropyridin-3,5-Dicarbaldehyd sowie den N,N'-substituierten Derivaten.

9. Verwendung nach einem der vorhergehenden Ansprüche, **dadurch gekennzeichnet, dass** der Gehalt an einer oder mehreren Substanzen aus der Gruppe der Lipofuscine zwischen 0,0001 und 30 Gew.-%, besonders vorteilhaft zwischen 0,01 und 10 Gew.-% gewählt wird, jeweils bezogen auf das Gesamtgewicht der Formulierung.

10. Verwendung einer oder mehrerer Substanzen, welche gewählt werden aus der Gruppe der Lipofuscine zur Herstellung einer kosmetischen oder dermatologischen Zubereitung nach Anspruch 5 oder 7, **dadurch gekennzeichnet, dass** die Zubereitung in Form einer Mehrfachemulsion, Mikroemulsion, Pickering-Emulsion oder sprühbarer Emulsion vorliegt.

11. Verwendung einer oder mehrerer Substanzen, welche gewählt werden aus der Gruppe der Lipofuscine zur Herstellung einer kosmetischen oder dermatologischen Zubereitung nach Anspruch 5 oder 7, **dadurch gekennzeichnet, dass** sie in Form einer Presun-, einer Sonnenschutz- oder einer Aftersunformulierung vorliegt.

## Claims

1. Use of one or more substances chosen from the group of lipofuscins for nontherapeutically tanning the skin.

2. Use of one or more substances chosen from the group of lipofuscins for nontherapeutically increasing the melanin synthesis in the skin.

3. Use of one or more substances chosen from the group of lipofuscins for nontherapeutically enhancing the existing tan of the skin.

4. Use of one or more substances chosen from the group of lipofuscins for nontherapeutically prolonging the brown coloration of the skin.

5. Use of one or more substances chosen from the group of lipofuscins for preparing a cosmetic or dermatological preparation for tanning the skin, for increasing the melanin synthesis, for enhancing the existing tan and/or for prolonging the brown coloration of the skin.

6. Use of one or more substances chosen from the group of lipofuscins for nontherapeutically protecting the skin against oxidative stress.

7. Use of one or more substances chosen from the group of lipofuscins for preparing a cosmetic or dermatological preparation for nontherapeutically protecting the skin against oxidative stress.

8. Use according to any of the preceding claims, **characterized in that** the lipofuscins are chosen from the group formed by 1-amino-3-iminopropene and from the N,N'-substituted derivatives and 1,4-dihydropyridine-3,5-dicarbaldehyde and the N,N'-substituted derivatives.

9. Use according to any of the preceding claims, **characterized in that** the content of one or more substances from the group of lipofuscins is between 0.0001 and 30% by weight, particularly advantageously between 0.01 and 10% by weight, in each case based on the total weight of the formulation.

10. Use of one or more substances chosen from the group of lipofuscins for preparing a cosmetic or dermatological preparation according to claim 5 or 7, **characterized in that** the preparation is in the form of a multiple emulsion, microemulsion, Pickering emulsion or sprayable emulsion.

11. Use of one or more substances chosen from the group of lipofuscins for preparing a cosmetic or dermatological preparation according to claim 5 or 7, **characterized in that** it is in the form of a presun formulation, a sunscreen formulation or an aftersun formulation.

## Revendications

1. Utilisation d'une ou plusieurs substances qui sont choisies dans le groupe des lipofuscines, pour le bronzage non thérapeutique de la peau.

2. Utilisation d'une ou plusieurs substances qui sont choisies dans le groupe des lipofuscines, pour l'augmentation non thérapeutique de la synthèse de la mélanine dans la peau.

3. Utilisation d'une ou plusieurs substances qui sont choisies dans le groupe des lipofuscines, pour l'accentuation non thérapeutique du bronzage présent de la peau.

4. Utilisation d'une ou plusieurs substances qui sont choisies dans le groupe des lipofuscines, pour le prolongement non thérapeutique de la coloration brune de la peau.

5. Utilisation d'une ou plusieurs substances qui sont choisies dans le groupe des lipofuscines, pour la fabrication d'une préparation cosmétique ou dermatologique destinée au bronzage de la peau, à l'augmentation de la synthèse de la mélanine, à l'accentuation du bronzage présent et/ou au prolongement de la coloration brune de la peau.

6. Utilisation d'une ou plusieurs substances qui sont choisies dans le groupe des lipofuscines, pour la protection non thérapeutique de la peau contre le stress oxydant.

7. Utilisation d'une ou plusieurs substances qui sont choisies dans le groupe des lipofuscines, pour la fabrication d'une préparation cosmétique ou dermatologique destinée à la protection non thérapeutique de la peau contre le stress oxydant.

8. Utilisation selon l'une quelconque des revendications précédentes, **caractérisée en ce que** la ou les lipofuscines sont choisies dans le groupe qui est constitué par le 1-amino-3-iminopropène ainsi que par les dérivés N,N'-substitués et le 1,4-dihydropyridine-3,5-dicarbaldéhyde ainsi que les dérivés N,N'-substitués.

9. Utilisation selon l'une quelconque des revendications précédentes, **caractérisée en ce que** la teneur en une ou plusieurs substances choisies dans le groupe des lipofuscines est choisie entre 0,0001 et 30 % en poids, de façon particulièrement avantageuse entre 0,01 et 10 % en poids, chaque fois par rapport au poids total de la composition.

10. Utilisation d'une ou plusieurs substances qui sont choisies dans le groupe des lipofuscines, pour la fabrication d'une préparation cosmétique ou dermatologique selon la revendication 5 ou 7, **caractérisée en ce que** la préparation se trouve sous forme d'une émulsion multiple, d'une microémulsion, d'une émulsion de Pickering ou d'une émulsion pulvérisable.

11. Utilisation d'une ou plusieurs substances qui sont choisies dans le groupe des lipofuscines, pour la fabrication d'une préparation cosmétique ou dermatologique selon la revendication 5 ou 7, **caractérisée en ce que** la préparation se trouve sous forme d'une composition avant-soleil, d'une composition antisolaire ou d'une composition après-soleil.
